# EUROPÄISCHE PATENTSCHRIFT

(11) **EP 0 818 191 B1**
(45) Veröffentlichungstag und Bekanntmachung des Hinweises auf die Patenterteilung: **17.03.1999**
(21) Anmeldenummer: 97111883.1
(22) Anmeldetag: 12.07.1997
(51) Int. Cl.: A61K 7/13

(54) **Haarfärbemittel**
Hairdying composition
Composition de coloration pour cheveux

(30) Priorität: 13.07.1996 DE 19628345
(43) Veröffentlichungstag der Anmeldung: 14.01.1998
(73) Patentinhaber: GOLDWELL GmbH, D-64297 Darmstadt (DE)
(72) Erfinder: Lorenz, Heribert, 64401 Gross-Bieberau (DE); Golinski, Frank, Dr., 64297 Darmstadt (DE)

(56) Entgegenhaltungen:
- EP-A- 0 766 958
- DE-A- 2 714 831
- DE-C- 4 301 663
- FR-A- 2 649 887

## Beschreibung

Die vorliegende Erfindung betrifft ein Haarfärbemittel auf Basis eines mit Peroxid reagierenden Entwickler-Kuppler-Systems, das einen ausdrucksvollen, brillanten und dauerhaften orange/kupferbraunroten Grundton liefert, der entweder als solcher angewandt werden, oder, in Kombination mit weiteren Entwickler- und/oder Kupplersubstanzen, zur Erzielung weiterer Farbnuancen benutzt werden kann.

Die nach wie vor in Haarfärbemitteln meist eingesetzten Entwicklersubstanzen sind 1,4-Diaminobenzol (p-Phenylendiamin) und 1-Methyl-2,5-diaminobenzol (p-Toluylendiamin). Die Verwendung dieser Substanzen ist insofern nicht völlig problemfrei, als sie bei extrem empfindlichen Personen in speziellen Fällen zu Hautsensibilisierungen führen können (bei sogenannten "Para-Allergikern").

Es wurde bereits versucht, dieses Problem durch Verwendung alternativer Entwicklersubstanzen zu lösen. Dies ist in beschränktem Umfang möglich durch den Einsatz von Tetraaminopyrimidin oder 1-(β-Hydroxyethyl)-2,5-diaminobenzol (vgl. EP-A 7537 und EP-B 400 330, in der Zulassungsliste für Haarfarben der EG unter der Nr. A 80 enthalten); jedoch müssen dann erhebliche Abstriche in der Färbeintensität und den Variationsmöglichkeiten der verschiedenen Farbtöne hingenommen werden.

Auch 1-Amino-4-bis-(2'-hydroxyethyl)aminobenzol (N,N-Bis-(2-hydroxyethyl)-p-phenylendiamin) und dessen Salze sind bereits als Entwicklersubstanzen in Haarfärbemitteln vorgeschlagen worden und in der vorläufigen Zulassungsliste für Haarfarben der EG unter der Nr. A 50 enthalten; jedoch bleiben auch bei Verwendung dieser Substanz in Kombination mit den üblichen Kupplersubstanzen noch farbtechnische Wünsche offen.

Schließlich ist auch 2- Amino- 3- hydroxypyridin als Kupplersubstanz in Haarfärbemitteln in Kombination mit einer Vielzahl möglicher Entwicklersubstanzen, darunter beispielsweise auch 1- Amino- 4- bis(2'- hydroxyethyl)aminobenzol, 1- (β- Hydroxyethyl)- 2,5- diaminobenzo\ und 4- Amino- 3- methylphenol bereits beschrieben (DE 43 01 663 C (GOLDWELL)).

Die Erfindung geht von der Aufgabenstellung aus, ein Haarfärbemittel zu schaffen, das 1-Amino-4-bis-(2'-hydroxyethyl)aminobenzol, und/oder 1-(β-Hydroxyethyl)-2,5-diaminobenzol bzw. deren wasserlösliche Salze als Entwicklersubstanz enthält und zur Herstellung eines orange/kupferbraunroten Farbtons geeignet ist.

Diese Aufgabe wird dadurch gelöst, daß ein solches Haarfärbemittel ein mit Peroxid reagierendes Entwickler-Kuppler-System enthält, das aus einer Kombination aus einer Entwicklersubstanz, enthaltend mindestens eine der Komponenten 1-Amino-4-bis-(2'-hydroxyethyl)aminobenzol und 1-(β-Hydroxyethyl)-2,5-diaminobenzol bzw. deren wasserlösliche Salze, 2-Amino-3-hydroxypyridin und 4-Amino-3-methylphenol besteht.

Bei Anwendung dieser Zusammensetzung auf Basis einer üblichen Grundlage wird nach der Oxidation mit Peroxid eine sehr ausdrucksvolle, intensive orangefarbene/kupferrotbraune Grundfärbung erhalten, die durch Zusatz entsprechender weiterer Kupplersubstanzen zu anderen, ebenso intensiven Farbnuancen variiert werden kann.

Weitere zusätzlich verwendbare Kupplersubstanzen sind insbesondere Resorcin, 2-Methylresorcin, 4-Chlorresorcin, 3-Aminophenol, 3-Amino-2-methylamino-6-methoxypyridin, 2-Aminophenol, 2-Amino-4-β-hydroxyethylaminoanisol bzw. dessen wasserlösliche Salze und α-Naphthol.

Damit soll jedoch der Zusatz weiterer Kupplersubstanzen keineswegs ausgeschlossen sein.

Auch die Mitverwendung weiterer, an sich bekannter Entwicklersubstanzen ist möglich. Neben den bereits eingangs genannten sind hierbei insbesondere noch 4-Aminophenol, 5-Aminosalicylsäure und/oder Hydroxytriaminopyrimidine, wie sie aus der EP-B 467 026 bekannt sind, zu erwähnen.

Als solche werden insbesondere das aus der bereits erwähnten EP-B 467 026 bekannte 2,4,5-Triamino-6-hydroxypyrimidin und das 4,5,6-Triamino-2-hydroxypyrimidin eingesetzt, vorzugsweise als Sulfat-Salze.

Die Konzentration der Entwicklersubstanzen liegt zwischen etwa 0,05 und 5 %, vorzugsweise 0,1 und 4 %, insbesondere 0,25 bis 0,5 % und 2,5 bis 3 % Gew.-% der Gesamtzusammensetzung des Haarfärbemittels (ohne Oxidationsmittel), wobei sich die Angaben jeweils auf den Anteil an freier Base beziehen.

Die Kupplersubstanzen als Reaktionspartner der Entwicklersubstanz(en) liegen in den erfindungsgemäßen Haarfärbemitteln etwa im gleichen Anteil wie die Entwicklersubstanzen vor, d.h., also in Mengen von 0,05 bis 5,0 %, vorzugsweise 0,1 bis 4 %, insbesondere 0,5 bis 3 Gew.-% der Gesamtzusammensetzung (ohne Oxidationsmittel), wobei sich die Angaben jeweils auf den Anteil an freier Base beziehen.

Das Gewichtsverhältnis von 4-Amino-3-methylphenol zu 2-Amino-3-hydroxypyridin liegt vorzugsweise bei 1:10 bis 10:1, insbesondere 1:5 zu 5:1, vor allem 1:2 zu 2:1, z.B. 1:1.

Auch hier ist es, wie bereits erwähnt, möglich und zuweilen auch zweckmäßig, weitere bekannte Kupplefsubstanzen mitzuverwenden, falls dies zur Erzielung bestimmter Farbnuancen erwünscht und erforderlich ist.

Die erfindungsgemäßen Zusammensetzungen können erwünschtenfalls auch sogenannte Nuanceure zur Feineinstellung des gewünschten Farbtones, insbesondere auch direktziehende Farbstoffe, enthalten.

Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5 %, insbesondere 0,1 bis 1 % Gew.-% der Farbzusammensetzung (ohne Oxidationsmittel).

Die erfindungsgemäßen Haarfärbemittel können die in solchen Mitteln üblichen Grund- und Zusatzstoffe, Konditioniermittel, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind. Sie können als Lösungen, Cremes, Gele oder auch in Form von Aerosol-Präparaten vorliegen; geeignete Trägermaterial-Zusammensetzungen sind aus dem Stand der Technik hinreichend bekannt.

Zur Applikation wird das erfindungsgemäße Oxidationsfarbstoff-Vorprodukt mit einem Oxidationsmittel vermischt. Bevorzugtes Oxidationsmittel ist Wasserstoffperoxid, beispielsweise in 2- bis 6-prozentiger Konzentration.

Es können jedoch auch andere Peroxide wie Harnstoffperoxid und Melaminperoxid eingesetzt werden.

Der pH-Wert des applikationsfertigen Haarfärbemittels, d. h. nach Vermischung mit Peroxid, kann sowohl im schwach sauren, d. h. einem Bereich von 5,5 bis 6,9, im neutralen als auch im alkalischen Bereich, d. h. zwischen pH 7,1 und 9,5 liegen.

Im folgenden werden verschiedene Ausführungsbeispiele zur Erläuterung der Erfindung gegeben.

| Grundlage | |
|---|---|
| Stearylalkohol | 8,0 (Gew.-%) |
| Kokosfettsäuremonoethanolamid | 4,5 |
| 1,2-Propandiolmono/distearat | 1,3 |
| Kokosfettalkoholpolyglykolether | 4,0 |
| Natriumlaurylsulfat | 1,0 |
| Ölsäure | 2,0 |
| 1,2-Propandiol | 1,5 |
| Na-EDTA | 0,5 |
| Natriumsulfit | 1,0 |
| Eiweißhydrolysat | 0,5 |
| Ascorbinsäure | 0,2 |
| Parfum | 0,4 |
| Ammoniak, 25%ig | 8,5 |
| Ammoniumchlorid | 0,5 |
| Panthenol | 0,8 |
| Wasser | @ 100,00 |

Die erfindungsgemäße Entwickler-Kuppler-Kombination wurde jeweils, unter entsprechender Verringerung des Wassergehalts, in diese Grundlage eingearbeitet.

Die Ausfärbungen erfolgten jeweils an Woll-Läppchen und Strähnen aus gebleichtem Menschenhaar, durch Aufbringen einer 1:1-Mischung aus Farbstoff-Vorprodukt und 6%iger Wasserstoffperoxid-Lösung und zwanzigminütiger Einwirkung bei Zimmertemperatur, folgendem Auswaschen und Trocknen.

Es wurden die folgenden Färbungen erzielt:

| | | |
|---|---|---|
| Beispiel 1: | 0,33 (Gew.-%) | 1-Amino-4-bis-(2'-hydroxyethyl) aminobenzolsulfat (A 50) |
| | 0,14 | 4-Amino-3-methylphenol |
| | 0,28 | 2-Amino-3-hydroxypyridin |
| Färbung: | Kräftiges, violett glänzendes Rotbraun. | |
| Beispiel 1a: | Weglassen von 2-Amino-3-hydroxypyridin führte zu einer nicht verwertbaren blassen beigegrauen Färbung. | |
| Beispiel 2: | 0,28 (Gew.-%) | 1-(β-Hydroxyethyl)-2,5-diaminobenzolsulfat (A 80) |
| | 0,14 | 4-Amino-3-methylphenol |
| | 0,25 | 2-Amino-3-hydroxypyridin |
| Färbung: | Intensives Orange-braun. | |
| Beispiel 2a: | Weglassen des 2-Amino-3-hydroxypyridins führte zu einer unbrauchbaren, blaßbraunen Färbung. | |
| Beispiel 3: | 0,28 (Gew.-%) | A 80 |
| | 0,14 | 4-Amino-3-methylphenol |
| | 0,12 | 2-Amino-3-hydroxypyridin |
| | 0,12 | Resorcin |
| Färbung: | Kräftiges Braun-orange. | |
| Beispiel 4: | 0,28 (Gew.-%) | A 80 |
| | 0,14 | 4-Amino-3-methylphenol |
| | 0,12 | 2-Amino-3-hydroxypyridin |
| | 0,12 | 3-Aminophenol |
| Färbung: | Kräftiges Kupfer-braunrot. | |
| Beispiel 5: | 0,28 (Gew.-%) | A 80 |
| | 0,14 | 4-Amino-3-methylphenol |
| | 0,10 | 2-Amino-3-hydroxypyridin |
| | 0,10 | Resorcin |
| | 0,10 | 3-Aminophenol |
| Färbung: | Intensives Orange-braun. | |
| Beispiel 6: | 0,28 (Gew.-%) | A 80 |
| | 0,14 | 4-Amino-3-methylphenol |
| | 0,12 | 2-Amino-3-hydroxypyridin |
| | 0,16 | α-Naphthol |
| Färbung: | Intensives Kupfer-rotbraun. | |

## Patentansprüche

1. Haarfärbemittel auf Basis eines mit Peroxid reagierenden Entwickler-Kuppler-Systems, dadurch gekennzeichnet, daß es eine Kombination aus
a) mindestens einer Verbindung, ausgewählt aus der Gruppe 1-Amino-4-bis-(2'-hydroxyethyl)aminobenzol und 1-(β-Hydroxyethyl)-2,5-diaminobenzol bzw. deren wasserlöslichen Salzen;
b) 2-Amino-3-hydroxypyridin; und
c) 4-Amino-3-methylphenol enthält.

2. Haärfärbemittel nach Anspruch 1, dadurch gekennzeichnet, daß es mindestens eine zusätzliche Kupplersubstanz, ausgewählt aus der Gruppe Resorcin, 2-Methylresorcin, 4-Chlorresorcin, 3-Aminophenol, α-Naphthol, 3-Amino-2-methylamino-6-methoxypyridin und/oder 2-Amino-4-(β-hydroxyethylamino)anisol bzw. dessen wasserlöslichen Salzen, enthält.

## Claims

1. Hair dyeing composition based on a developing/coupling agent system reacting with peroxides, comprising a combination of
a) at least one compound selected from the group 1-amino-4-bis-(2'-hydroxyethyl)aminobenzene and 1-(β-hydroxyethyl)-2,5-diaminobenzene, or the water-soluble salts thereof,
b) 2-amino-3-hydroxypyridine; and
c) 4-amino-3-methylphenol.

2. Hair dyeing composition according to claim 1, comprising at least one additional coupling agent, selected from the group of resorcinol, 2-methyl resorcinol, 4-chlororesorcinol, 3-aminophenol, α-naphthol, 3-amino-2-methyl amino-6-methoxypyridine and (or) 2-amino-4-(β-hydroxyethyl amino) anisole or the water-soluble salts thereof.

## Revendications

1. Agent colorant capillaire à base d'un système révélateur/coupleur réagissant avec un peroxyde, caractérisé en ce qu'il contient une combinaison de :
a) au moins un composé, choisi dans le groupe formé par le 1-amino-4-bis-(2'-hydroxyéthyl)aminobenzène et le 1-(β-hydroxyéthyl)-2,5-diaminobenzène ou bien leurs sels hydrosolubles;
b) la 2-amino-3-hydroxypyridine; et
c) le 4-amino-3-méthylphénol.

2. Agent colorant capillaire selon la revendication 1, caractérisé en ce qu'il contient au moins une substance de couplage supplémentaire, choisie dans le groupe formé par la résorcine, la 2-méthylrésorcine, la 4-chlororésorcine, le 3-aminophénol, l'α-naphthol, la 3-amino-2-méthylamino-6-méthoxy-pyridine et/ou le 2-amino-4-(β-hydroxyéthylamino)anisol ou bien leurs sels hydrosolubles.
